# EUROPÄISCHE PATENTSCHRIFT

(11) **EP 1 545 781 B1**
(45) Veröffentlichungstag und Bekanntmachung des Hinweises auf die Patenterteilung: **31.10.2012**
(21) Anmeldenummer: 03798178.4
(22) Anmeldetag: 23.09.2003
(51) Int. Cl.: B01L 3/00, B81B 1/00, A61J 1/00, A61M 1/00, A61M 39/00

(54) **TRÄGERELEMENT FÜR DIAGNOSTISCHE TESTS**
SUPPORTING ELEMENT FOR CONDUCTING DIAGNOSTIC TESTS
ELEMENT DE SUPPORT POUR ANALYSES DIAGNOSTIQUES

(30) Priorität: 23.09.2002 DE 10244154
(43) Veröffentlichungstag der Anmeldung: 29.06.2005
(73) Patentinhaber: Medion Diagnostics AG, 3186 Düdingen (CH)
(72) Erfinder: SCHWIND, Peter, 1700 Fribourg (CH); BECKER, Holger, 07743 Jena (DE)
(74) Vertreter: Heselberger, Johannes
(86) Internationale Anmeldenummer: PCT/EP2003/010590
(87) Internationale Veröffentlichungsnummer: WO 2004/028692

(56) Entgegenhaltungen:
- EP-A- 0 073 847
- EP-A- 1 048 306
- WO-A-99/46045
- US-A1- 2002 053 532
- US-B1- 6 418 968

## Beschreibung

Die Erfindung betrifft ein Trägerelement zur Durchführung von diagnostischen Tests, insbesondere Agglutinationsbestimmungen mit mindestens einem Unterteil, mindestens einem Oberteil und mindestens einem zentralen Zulauf, wobei von dem zentralen Zulauf mindestens zwei Zuleitungskanäle zu jeweils einer Reaktionskammer führen, sowie ein System zur Agglutinationsbestimmung sowie ein System zur einfachen Durchführung von Blutgruppen-Bedside-Tests.

Aus der WO 99/46045 ist ein Probenträger mit mehreren Probenaufnahmekammern für eine Probenflüssigkeit bekannt. Von diesen Probenaufnahmekammern erstreckt sich jeweils ein Verteilerkanal für Probenflüssigkeit, von dem wiederum mehrere Zulaufkanäle abzweigen, die ihrerseits jeweils in einer Reaktionskammer münden. Dabei sind die Verteilerkanäle und Zulaufkanäle derart ausgestaltet, dass der Flüssigkeitstransport durch die Kanäle aufgrund von Kapillarkräften erfolgt. Weiterhin ist in jeder Reaktionskammer im Einmündungsbereich eines Zulaufkanals eine Einrichtung angeordnet, die zur Erzeugung einer Kapillarkraft zum Fließen der Probenflüssigkeit aus dem Zulaufkanal in die Reaktionskammer dient. Zur Durchführung von Tests muss hier die Probenaufnahmekamner mit der Testflüssigkeit gefüllt werden, damit von der Probenaufnahmekammer aus eine sukzessive Verteilung der Flüssigkeit in die verschiedenen Reaktionskammern erfolgen kann. Dies führt dazu, dass sich erst nach einer gewissen Zeit ein Gleichgewicht zwischen der Probenaufnahmekammer und den in Serie geschalteten Reaktionskammern einstellt. Folglich starten die gewünschten Reaktionen in den Reaktionskammern zu unterschiedlichen Zeitpunkten, was gerade bei der Durchführung von Schnelltests, insbesondere Blutgruppenschnelltests zu unerwünschten Verzögerungen und unter Umständen auch zu falschen Ergebnissen führen kann.

Das Gebrauchsmuster DE 295 00 587 U1 offenbart einen Bausatz zur Blutgruppenbestimmung mit einer Testeinheit, die aus mehreren verschweißten Flächen besteht, wobei im Innern ein Kanalsystem ausgebildet ist, wobei sich die einzelnen Kanäle von Kompartimenten aus, in denen die entsprechenden Reaktionen stattfinden sollen, über einen Ventilmechanismus in einen gemeinsamen Zulauf erstrecken. Über den Ventilmechanismus werden sämtliche Kompartimente erreicht, wobei die Kompartimente einseitig mit einer elastischen Folie verschweißt bzw. verklebt sind. Um das Ventil zu öffnen, muss ein starker Überdruck angelegt werden. Ein Nachlassen des Druckes wird zum Rückfließen der Flüssigkeiten führten, wenn innerhalb des Systems genügend Druck aufgebaut ist, um die Ventile dadurch zu öffnen. Eine Befüllung ist nur möglich, da dehnbare Folien zum Verschließen der Einheit genutzt werden. Aufgrund dieser Beschaffenheit werden die Reaktionsgefäße ungleichmäßig mit Flüssigkeit befüllt. Außerdem ist bei diesem Bausatz ein Austreten von Lösungen aus den Reaktionsgefäßen möglich.

Aufgabe der Erfindung ist es, die im Hinblick auf den Stand der Technik angeführten Nachteile zu überwinden. Des Weiteren soll ein möglichst einfach zu fertigendes und kostengünstig zu produzierendes Trägerelement zur Agglutinationsbestimmung bereitgestellt werden, mit dem Agglutinationstests einfacher und sicherer durchgeführt werden können.

Die Aufgabe wird erfindungsgemäß gelöst durch ein Trägerelement zur Agglutinationsbestimmung mit mindestens einem Unterteil, mindestens einem Oberteil und mindestens einem zentralen Zulauf, wobei von dem zentralen Zulauf mindestens zwei Zuleitungskanäle zu jeweils einer Reaktionskammer führen, wobei die mindestens zwei Zuleitungskanäle jeweils gleiche Volumina aufweisen und zwischen dem mindestens einen zentralen Zulauf und den Reaktionskammern jeweils eine Ventilfunktion angeordnet ist.

Durch die erfindungsgemäße Ausgestaltung der Zuleitungskanäle können Agglutinationstests, insbesondere Blutgruppenschnelltests einfacher und sicherer durchgeführt werden, da alle Reaktionen, nämlich die Hämagglutination von Erythrozyten, in den Kammern gleichzeitig mit dem jeweils gleichen Probenvolumen pro Zeiteinheit ablaufen. Durch das gleiche Volumen und den gleichen Startzeitpunkt kann die Parallelität der Vorrichtung wirklich genutzt werden. Das Personal muss also nicht unnötig lange auf Ergebnisse warten, darüber hinaus wird auch die Gefahr einer falschen Auswertung aufgrund zeitlich verzögerter Reaktionen gebannt. Das vorstehend Beschriebene lässt deutlich erkennen, dass durch die Anwendung einfacher konstruktiver Maßnahmen eine notwendige Verminderung der Fehleranfälligkeit von Agglutinationstest, insbesondere Blutgruppenschnelltests erzielt wird. Die Tests können schneller und einfacher durchgeführt werden, es werden weniger Schritte benötigt und die Gefahr von Bedienungsfehlern wird wesentlich vermindert. Das erfindungsgemäße Trägerelement ist besonders geeignet für solche Schnelltests zur Überprüfung der Blutgruppen A, B und O bzw. A, B, O und D bei Empfänger und Blutbeutel, die einschließlich vollständiger Dokumentation direkt vor der Transfusion durch das transfundierende Personal an der Patienten-Bettseite im Rahmen sogenannter Bedside-Tests durchgeführt werden.

Erfindungsgemäß sind die Ventile den Reaktionskammern vorzugsweise direkt vorgeschaltet, so dass sowohl Kreuzkontaminationen verhindert werden als auch jeglicher Rückfluss von Probenflüssigkeit aus der Reaktionskammer in die Zuleitungskanäle ausgeschlossen wird. Es wird außerdem vermieden, dass es zu ungewollten Reaktionen bereits im Kanal kommt, wodurch Verstopfungen des Systems und Fehlinterpretationen unterbunden werden. Aufgrund der erfindungsgemäßen Zuleitungskanäle unterbunden passive Kapillarventile eingesetzt. Dies hat zur Folge, dass keine beweglichen Komponenten bzw. dehnbare Folien oder zerreißbare Membranen notwendig sind, wodurch die Störanfälligkeit der erfindungsgemäßen Schnelltests geringer ist. Unter fertigungstechnischen Gesichtspunkten ist diese Ausführungsform auch erheblich günstiger.

In einer bevorzugten Ausführungsform der Erfindung weisen die Zuleitungskanäle sowohl die gleiche Länge als auch den gleichen Durchmesser auf. Dadurch wird auf noch einfachere Weise die erforderliche gleichmäßige Befüllung der Reaktionskammern erreicht.

Zur Erzielung des gleichen Volumens können die Zuleitungskanäle in der Länge und im Durchmesser variiert werden, allerdings muss dann entsprechend der unterschiedliche Druckabfall in die Berechnung mit einbezogen werden. Die Länge eines Zuleitungskanals wird gemessen von dem Punkt, an dem der Kanal vom zentralen Zulauf abzweigt, bis zu der jeweiligen Reaktionskammer oder bis zu einer evtl. vorhandenen Ventilfunktion vor der Reaktionskammer. Zur Berechnung des Kanalvolumens wird diese Länge dann mit dem (Innen-) Durchmesser des Kanals multipliziert.

Vorzugsweise ist mindestens einer der Zuleitungskanäle mäanderförmig ausgebildet. Die Mäander stellen eine Verlängerung der Kanäle dar, die in erster Linie als Gaspuffer wirkt, um auf eine effiziente Weise das Austreten von Probenflüssigkeit aus den Reaktionskammern zurück in die Zuleitungskanäle zu verhindern. Dadurch können Verstopfungen des Systems und daraus resultierende Fehlinterpretationen unterbunden werden.

Es besteht darüber hinaus auch die Möglichkeit, zwei hintereinandergeschaltete Ventile vorzusehen. Eine solche Doppelventilfunktion bietet den Vorteil, dass der Gesamtwiderstand gegen den Austritt von Flüssigkeit aus den Reaktionskammern erhöht werden kann.

In einer weiteren bevorzugten Ausführungsform weisen die Reaktionskammern jeweils einen Lüftungskanal auf. Der Lüftungskanal führt dabei aus der Reaktionskammer hinaus, so dass in der Reaktionskammer befindliche Luft von der einströmenden Probenflüssigkeit verdrängt werden kann. Die Lüftungskanäle dienen, vor allem bei kleiner Dimensionierung, gleichzeitig als Gaspuffer, der ein Austreten von Lösungen aus der Reaktionskammer in den Lüftungskanal selbst verhindert. Um einen noch weitgehenderen Schutz vor Flüssigkeitsaustritt in die Lüftungskanäle zu bieten, können zwischen Reaktionskammern und Lüftungskanälen jeweils Ventilfunktionen angeordnet sein. Die Lüftungskanäle verfügen vorzugsweise über einen Ausgang aus dem Trägerelement, der vor der Benutzung beispielsweise mit einer Folie verschlossen sein kann.

In einer noch bevorzugteren Ausführungsform ist der Deckel über jeder Reaktionskammer des Trägerelements mit mindestens einer Öffnung versehen, durch die die Entlüftung erfolgt. Dies ist die direkteste und zugleich schnellste Möglichkeit, die in den Reaktionskammern befindliche Luft entweichen zu lassen und stellt zudem eine signifikante konstruktive Vereinfachung gegenüber den aufwendigeren Lüftungskanälen mit Ventilfunktionen dar. Die Öffnungen sind vorzugsweise rund und weisen vorzugsweise einen Durchmesser von 1, 5 mm oder weniger auf, so dass selbst wenn das Trägerelement stark geschüttelt oder auf den Kopf gestellt wird, es zu keinem Austritt von Flüssigkeit aus der Kammer nach außen kommen kann. Es handelt sich also nach wie vor um ein weitestgehend geschlossenes System.

Vorzugsweise werden die, in der Regel jeweils aus Kunststoff gefertigten, Unter- und Oberteile dicht zusammengefügt, beziehungsweise verbunden, nachdem die Reaktionskammern mit Antikörpern oder Antigenen, den sogenannten Reagenzien, beschickt worden sind. Dies kann, je nach gewünschter Fertigungstechnik, beispielsweise durch Zusammenstecken oder Bonden bzw. Kleben erfolgen. Es sind aber auch andere Verbindungstechniken denkbar. Das fertige Trägerelement ist vorzugsweise ein geschlossenes durchsichtiges Plastikgehäuse, damit die Befüllung und im Regelfall auftretende Agglutinationsreaktionen vom Bedienpersonal beobachtet werden können. Vorzugsweise werden die Entlüftungsöffnungen erst bei Nutzung der Reaktionsgefäße, vorzugsweise durch das Abziehen einer Folie geöffnet. Der Zulauf wird vorzugsweise auch erst bei der Nutzung des Trägerelements geöffnet, um zum Beispiel ein Aufstecken bzw. Aufschrauben von Luer-Lok-Spritzen zu ermöglichen. Bei dem Trägerelement handelt es sich vorzugsweise vor der Nutzung um ein geschlossenes System, wodurch ein Verdunsten und eine Kontamination der Lösungen in den Reaktionskammern verhindert wird und was einem Austreten der Lösungen entgegenwirkt. Die Reaktionskammern enthalten vorzugsweise spezifische Antikörper, die vorzugsweise gegen die Blutgruppenmerkmale A und B bzw. A, B und D gerichtet sind.

Für eine Blutgruppenbestimmung, insbesondere einen Bedside-Test, können die Trägerelemente so ausgestaltet sein, dass sich auf einer Seite ein Zulauf für eine Blutprobe aus dem Blutbeutel (Spenderseite S) und auf der anderen Seite ein Zulauf für eine Blutprobe des Patienten (Empfängerseite E) befindet. Dadurch werden die korrespondierenden Testergebnisse, ohne dass eine Verwechslungsgefahr besteht, gegenüber gestellt. Natürlich kann das Trägerelement auch nur einen Zulauf, für die Bestimmung lediglich einer der beiden Seiten, aufweisen. Als mögliche Trägerelementkonfigurationen kommen daher in Frage ein ABO-Element für Spender und/oder Empfänger bzw. ein ABOD-Element für Spender und/oder Empfänger. Diejenigen Trägerelemente, die spender- und empfängerseitig eingesetzt werden, können über eine Sollbruchstelle trennbar miteinander verbunden sein, falls beispielsweise nur Empfängerseitig getestet werden soll, so dass dann zwei Empfänger mit einem Trägerelement 1 getestet werden können. Außerdem kann eine Steckoption vorgesehen sein, mit der zum Beispiel bei mehreren hintereinander durchgeführten Transfusionen die entsprechenden Tests miteinander verbunden werden können.

Die Antikörper oder Antigene in den Reaktionskammern liegen vorzugsweise in gelöster Form als Reagenzienlösung vor. Die Reagenzien können aber auch in lyophilisierter oder an eine Festphase gebundener Form, also etwa an Wände bzw. Böden der Reaktionskammern oder an synthetische Partikel gebunden bzw. beschichtet (gecoated), vorliegen. Vorzugsweise werden im Rahmen der Erfindung organische Polymerpartikel verwendet. Diese sind vorteilhafterweise farblos oder rot. Ein besonders bevorzugtes Material ist dabei Polystyrol. Die Robustheit dieser Partikel ermöglicht eine Immobilisierung von Antikörpern nach bekannten industriellen Standardmethoden auf ihrer Oberfläche. Die Antikörpermoleküle können über adsorptive, kovalente oder hochaffine Wechselwirkungen immobilisiert werden. Die kovalente Kopplung kann beispielsweise über chemisch reaktive Gruppen, die auf der Oberfläche der Partikel exponiert sind, erfolgen. Beispiele für solche Gruppen sind Carboxyl-, Amino-, Aldehyd-, und Epoxygruppen. Die Immobilisierung über hochaffine Wechselwirkungen wird durch zwei Partner eines hochaffinen Bindepaares vermittelt, wie etwa Streptavidin bzw. Avidin mit Biotin. Die Immobilisierung kann direkt unter Verwendung von Linkem erfolgen, um eine bevorzugte optimale Orientierung der gebundenen Antikörpermoleküle zu erzielen. Als Agglutinationsreagenz werden bei einem Blutgruppentest die antikörpergebundenen synthetischen Partikel und das Blut des Spenders oder Empfängers verwendet. Bei einer positiven Reaktion von beispielsweise Blutgruppe-A-Erythrozyten mit Polymerpartikeln, die anti-A-Antikörper gebunden haben, entstehen vernetzte Agglutinationskomplexe aus farblosen bzw. roten Partikeln und natürlicherweise rot gefärbten Erythrozyten, die als rote Agglutinate erkennbar sind. Eine solche Agglutination zeigt eine positive Reaktion an.

In einer bevorzugten Ausführungsform weisen die Reaktionskammern eine badewannenartige Form auf, d.h. die Seitenwände und die Stirnseiten der Reaktionskammern fallen zunächst mit leichter Neigung ab, um dann in den Boden der Reaktionskammer überzugehen. Wichtig ist dabei, dass der Übergang von den Seitenwänden zum Boden jeweils abgerundet ist, damit eine gleichmäßige Verteilung der Reagenzienlösung in der Reaktionskammer sichergestellt werden kann. Eine derartige Badewannenform sorgt für eine schnellere Durchmischung der Reaktanden, was eine schnellere und stärkere Reaktion zur Folge hat. Dadurch wird dem Bedienpersonal das Ablesen der Testergebnisse leichter gemacht. In alternativen bevorzugten Ausführungsformen sind die Reaktionskammern halbkugelförmig oder als Halbellipsoid ausgestaltet. Dadurch wird ein ähnlicher Effekt erzielt.

Die in den Reaktionskammern befindliche Reagenzienlösung wird vorzugsweise durch Zugabe einer zusätzlichen Lösung verdichtet. Hierfür kommt vorzugsweise Glycerin, vorzugsweise mit einer Konzentration zwischen 5 und 30 % w/v (Gewichtsprozent) zum Einsatz. Glycerin weist eine höhere Dichte auf als die Antikörperlösung, wodurch sich die Viskosität der Reagenzienlösung erhöht. Diese Maßnahme dient wiederum der Stabilität, da eine solche Lösung mit höherer Viskosität die Ventile noch schwerer durchbricht als die unverdichtete Reagenzienlösung. Außerdem hat das Glycerin in dem angegebenen Konzentrationsbereich keinen ungünstigen Einfluss auf die Reaktivität oder Avidität der Antikörper oder Antigene und beeinflusst darüber hinaus auch nicht die Stabilität oder Funktionsweise der Erythrozyten. Erfindungsgemäß können außer Glycerin auch andere geeignete viskositätserhöhende Lösungen zur Anwendung kommen.

Die Abstände der Reaktionskammern entsprechen vorzugsweise dem Titerplattenraster und weiter vorzugsweise dem einer 96-Well-Mikrotiterplatte, damit die Kammern automatisch mit handelsüblichen Mikrotiter-Pipettierrobotern befüllt werden können.

In einer bevorzugten Ausführungsform der Erfindung ist der mindestens eine zentrale Zulauf so ausgestaltet, dass an ihm ein Luer- oder Luer-Lok-Anschluss anordenbar ist. Zu diesem Zweck weist der zentrale Zulauf entweder eine entsprechende Steckvorrichtung oder ein entsprechendes Schraubgewinde auf.

Schließlich wird erfindungsgemäß ein System zur Agglutinationsbestimmung, insbesondere zur einfachen Durchführung von Blutgruppen-Bedside-Tests mit einem Trägerelement und einem Blutbeutel, bei dem zwischen dem Trägerelement und dem Blutbeutel eine Verbindung hergestellt wird, beansprucht. Im Rahmen der Erfindung kann die Kopplung des Systems aus Trägerelement und Blutbeutel auf drei verschiedene Arten erfolgen. In der ersten Variante wird das Trägerelement über einen Klammer- oder Steckmechanismus an den Blutbeutel geheftet und ist mit einer Luer- oder Luer-Lok-Spritze mit Kanüle versehen. In der bevorzugten zweiten Variante ist das Trägerelement über eine zusätzliche Schlauchverbindung mit dem Blutbeutel verbunden, wobei die Schlauchverbindung eine Kunststoffkanüle an ihrem blutbeutelseitigen Ende, einen Luer- oder Luer-Lok-Adaptor an ihrem trägerelementseitigen Ende und einen Schlauch zwischen den beiden Enden, aufweist. Bei der Schlauchverbindung handelt es sich vorzugsweise um ein Infusionsbesteck. Mit Hilfe dieser Variante kann insbesondere das Kontaminations- und Infektionsrisiko für das Bedienpersonal praktisch ausgeschlossen werden. In der besonders bevorzugten dritten Variante weist das für die Transfusion verwendete Transfusionsbesteck eine Verzweigung, beispielsweise ein sogenanntes Y-Verbindungsstück auf, mit deren Hilfe eine kleine Menge der Erythrozyten der Konserve direkt in das Trägerelement abgezweigt werden kann. Eine Rückflusssperre in dem Y-Verbindungsstück oder in dem Schlauchabschnitt zwischen dem Y-Verbindungsstück und dem Trägerelement verhindert dabei einen Rückfluss von Erythrozyten in den Transfusionsstrom. Auch mit dieser Variante kann das Kontaminations-und Infektionsrisiko für das Bedienpersonal praktisch ausgeschlossen werden.

Gegenstand der Erfindung ist des Weiteren ein System mit einem Schlauchsegmentöffner und mit dem erfindungsgemäßen Trägerelement. Herkömmliche Schlauchsegmentöffner, wie sie beispielsweise von der Firma Sarstedt hergestellt werden, umfassen ein fingerhutartiges d. h. leicht konisches und innen hohles Aufnahmeteil für Schlauchsegmente, die beispielsweise Blut enthalten können. In diesem Aufnahmeteil befindet sich eine spitze Nadel, mit der ein in das Aufnahmeteil eingeführtes Schlauchsegment angestochen werden kann. Mit Hilfe dieser Nadel kann die in dem Schlauchsegment enthaltene Flüssigkeit, also beispielsweise Blut, gegebenenfalls durch gezieltes Drücken auf das Schlauchsegment, in das dafür vorgesehene Plastikröhrchen transportiert werden. Die Nadel ist so in dem Aufnahmeteil angeordnet, dass sich das Bedienpersonal daran nicht verletzen kann. Bei dem Im Stand der Technik bekannten Schlauchsegmentöffner schließt sich an das Aufnahmeteil ein Endstück an, welches bis zum Anschlag des Aufnahmeteils in ein Plastikröhrchen für die Aufnahme des Bluts aus dem Schlauchsegment eingeführt werden kann, d. h. der Innendurchmesser des schmaleren Endes des Aufnahmeteils, an den sich das Endstück anschließt, ist grösser oder gleich dem Außendurchmesser des Plastikröhrchens.

Erfindungsgemäß wird das Endstück des Schlauchsegmentöffners als Luer-Lok Male Anschluss oder als Luer Male Anschluss ausgebildet. Mit Hilfe dieses erfindungsgemäßen Schlauchsegmentöffners wird das Zuführen des Bluts aus dem Schlauchsegment zu dem Trägerelement wesentlich vereinfacht. Durch den Luer-Lok Male Anschluss ist der Schlauchsegmentöffner mit einem Luer-Lok Female Anschluss des erfindungsgemäßen Trägerelements kompatibel. Diese Konstruktion des Schlauchsegmentöffners bietet den Vorteil, dass der Schlauchsegmentöffner und das Trägerelement fest miteinander gekoppelt werden können. Eine solche Konstruktionsweise kommt dem Bedienpersonal entgegen, da sie ein sauberes Ausdrücken des Schlauchsegments erleichtert. Früher musste das Bedienpersonal drei Gegenstände das Schlauchsegment, den Schlauchsegmentöffner und das Trägerelement halten, um dem Trägerelement Blut aus dem Schlauchsegment zuführen zu können. Hierfür musste das Bedienpersonal während dem Öffnen des Schlauchsegments mit Hilfe des Schlauchsegmentöffners zusätzlich das Trägerelement so unter den Schlauchsegmentöffner halten, dass das Blut aus dem Schlauchsegmentöffner in die dafür vorgesehenen Öffnung des Trägerelements tropft. Mit Hilfe des erfindungsgemäßen Schlauchsegmentöffners ist das Trägerelement fest mit dem Schlauchsegmentöffner verbunden. Das Bedienpersonal kann dadurch mit der einen Hand das Schlauchsegment festhalten und mit der anderen Hand den Schlauchsegmentöffner mit dem daran befestigten Trägerelement. Zum Zuführen des Bluts aus dem Schlauchsegment zu dem Trägerelement, muss das Bedienpersonal jetzt nur noch das Schlauchsegment in das Aufnahmeteil des Schlauchsegmentöffners einführen. Dadurch gelangt automatisch das aus dem Schlauchsegment austretende Blut direkt zu dem Trägerelement. Durch den erfindungsgemäßen Schlauchsegmentöffner wird dadurch das Zuführen von Blut zu dem Trägerelement vereinfacht und das Kontaminationsrisiko wesentlich verringert.

Im Folgenden wird ein Ausführungsbeispiel der Erfindung anhand der beigefügten Zeichnungen erläutert. Es zeigen:
- Fig. 1: eine Draufsicht auf eine bevorzugte Ausführungsform eines erfindungsgemäßen Trägerelements mit jeweils zwei Reaktionskammern auf Spender- und Empfängerseite;
- Fig. 2: einen Querschnitt durch das in Fig. 1 dargestellte erfindungsgemäße Trägerelement entlang der Linie A-A;
- Fig. 3: eine Draufsicht auf eine weitere bevorzugte Ausführungsform eines erfindungsgemäßen Trägerelements mit jeweils zwei Reaktionskammern auf Spender- und Empfängerseite;
- Fig. 4a: eine Draufsicht auf eine bevorzugte Ausführungsform einer Reaktionskammer;
- Fig. 4b: eine Schnittansicht entlang der Linie A - A in Fig. 4a;
- Fig. 4c: eine Schnittansicht entlang der Linie B -B in Fig. 4a;
- Fig. 5: ein erfindungsgemäßes System aus Trägerelement, Blutbeutel und Schlauchverbindung;
- Fig. 6: ein erfindungsgemäßes System aus Trägerelement, Blutbeutel und einer Schlauchverbindung mit einer Verzweigung.

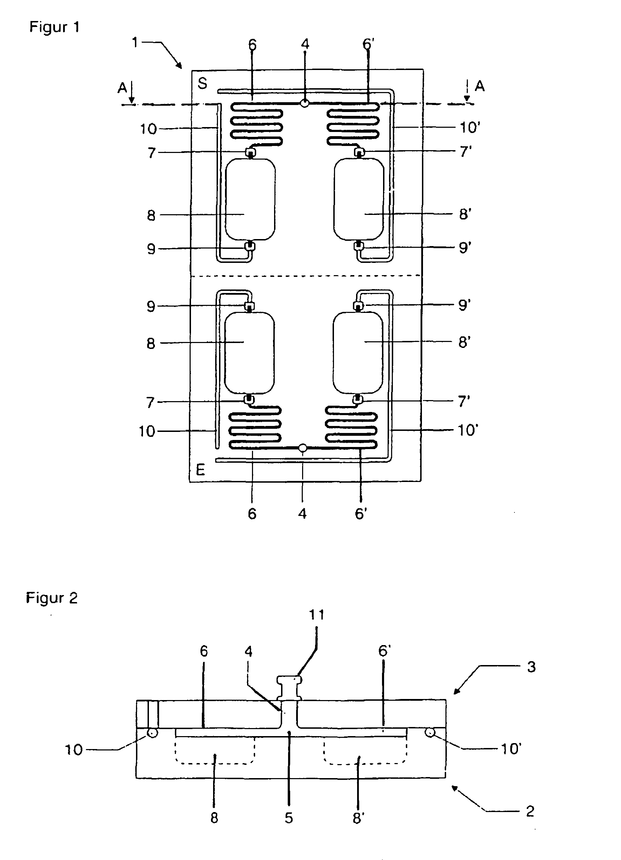

In Fig. 1 wird eine beispielhafte Ausführungsform eines Trägerelements 1 zur Agglutinationsbestimmung, insbesondere zur Blutgruppenbestimmung gezeigt. Diese ist so konfiguriert, dass auf der einen Seite eine Blutprobe aus einem Blutbeutel (Spenderseite S) und auf der anderen Seite eine Blutprobe von einem Patienten (Empfängerseite E) zugeführt werden kann. Beide Seiten unterscheiden sich konstruktiv nicht und sind somit grundsätzlich austauschbar. Im Folgenden beschränkt sich die Beschreibung deshalb auf eine Seite des Trägerelements.

Im vorliegenden Beispiel erstrecken sich von dem zentralen Zulauf 4 jeweils zwei mäanderförmige Zuleitungskanäle 6, 6' über die Ventilfunktionen 7,7' in die Reaktionskammern 8, 8'. Die Zuleitungskanäle 6, 6' weisen in dieser bevorzugten Ausführungsform die gleiche Länge und den gleichen Durchmesser auf. Von jeder Reaktionskammer 8, 8' zweigt ein Lüftungskanal 10, 10' nach außen ab, der ermöglicht, dass beim Einspritzen von Blut/Erythrozyten kein Rückdruck entsteht, indem Luft nach außen entweichen kann. Dem Entlüftungskanal vorgeschaltet sind die Ventilfunktionen 9, 9'. Die Ventilfünktionen 7, 9, 7, 9' sind in der Regel als passive Kapillarventile ausgebildet. Auch bei unterschiedlichen Kanalbreiten auf Einfüllseite und Entlüftungsseite können im vorliegenden Beispiel die gleichen Ventile verwendet werden.

Die Anordnung der Mäander und der Reaktionskammern 8, 8' ist in diesem Beispiel spiegelsymmetrisch, was ebenfalls dazu beiträgt, dass in den Zuleitungskanälen 6, 6' identische Strömungsbedingungen herrschen. Identische Strömungsbedingungen stellen eine bevorzugte Möglichkeit dar, das geforderte zeitgleiche Befüllen der Reaktionskammern 8, 8' mit identischen Volumina pro Zeiteinheit zu erreichen. Zudem sind die Abstände zwischen den Reaktionskammern 8, 8' so ausgelegt, dass sie mit denen gängiger Mikrotiterplatten übereinstimmen, damit für die produktionsmäßige Befüllung der Reaktionskammern 8, 8' des Unterteils 2 mit Antikörper-oder Antigenlösungen handelsübliche automatische Pipettierstationen verwendet werden können. Die Reaktionskammern 8, 8' sind vorzugsweise rund oder oval ausgebildet und fassen ein Volumen von 10 bis 1000 µl; besonders bevorzugt wird ein Volumen von 100 µl bis 500 µl. Die Zuführung der Fluide erfolgt in der vorliegenden Ausführungsform der Erfindung durch Druckbeaufschlagung, wobei die Verwendung von Kapillarkräften zur Zuführung der Fluide grundsätzlich auch möglich ist. Das Trägerelement kann außerdem zwischen Spender- und

Empfängerseite eine Sollbruchstelle (gestrichelt eingezeichnet) aufweisen, falls beispielsweise nur Empfängerseitig getestet werden soll, so dass dann zwei Empfänger mit einem Trägerelement 1 getestet werden können.

Ein Rückfluss der Antikörper aus den Reaktionskammern 8, 8' wird, wie bereits oben erwähnt, durch das erfindungsgemäße Design des Fluidsystems des Trägerelements 1 verhindert. Für die Dimensionierung des Fluidsystems sind neben dem Kriterium der gleichmäßigen Befüllung der Reaktionskammern und der Vermeidung des Austretens von Flüssigkeit aus der Reaktionskammer, die folgenden Punkte relevant: Befülldauer, Dosiergenauigkeit sowie Volumina und Beschaffenheit der Flüssigkeit in der Reaktionskammer und der zuzuführenden Flüssigkeiten. Dabei wird für die jeweilige Anwendung jeweils eine entsprechende Dimensionierung des Fluidsystems realisiert, wobei die Zuführung, Entlüftung als auch die Reaktionskammern in der Größenordnung von einigen Mikrometern bis einigen Millimetern liegen.

Fig. 2 zeigt einen Querschnitt durch Oberteil 3 und Unterteil 2 des erfindungsgemäßen Trägerelements 1 entlang der Linie A-A. Im Oberteil 3 befindet sich der zentrale Zulauf 4, der die Befüllung des geschlossenen Systems mit Proben ermöglicht. Das Oberteil 3 wirkt in dieser bevorzugten Ausführungsform als Deckel für die Kanäle und die Reaktionskammern 8, 8'. In einer hier nicht gezeigten Ausführungsform können die Kanäle und die Kammern allerdings auch vollständig im Innern des Unterteils angeordnet sein und über Kanäle mit den Reagenzien befüllt werden. Der zentrale Zulauf 4 kann zum Aufstecken oder Aufschrauben von handelsüblichen Spritzen oder Plastikpasteurpipetten ausgebildet sein und hat einen bevorzugten Durchmesser von 0,1 - 10 mm und einen weiter bevorzugten Durchmesser von 1 - 5 mm. In einer besonders bevorzugten Ausführung ist an dem zentralen Zulauf 4 ein Luer-Lok-Female-Anschluss 11 angeschraubt oder gesteckt beziehungsweise direkt in den Deckel integriert, der mit den handelsüblichen Luer-Spritzen und insbesondere mit den jeweiligen Luer-Lok-Spritzen kompatibel ist. Von dem zentralen Zulauf 4, zweigen die Zuleitungskanäle 6, 6' ab, deren Länge bis zu der der Reaktionskammer vorgeschalteten Ventilfunktion 7, 7' vom Punkt 5 aus bestimmt wird. Gestrichelt gezeigt sind auch die hinter dem zentralen Zulauf 4 und den mäanderförmigen Zuleitungskanälen 6, 6' liegenden Reaktionskammern 8, 8'. An den Seiten sind die Entlüftungskanäle 10, 10' zu sehen. Der Querschnitt von Fig. 2 veranschaulicht dabei, wo der Entlüftungskanal 10 in diesem Beispiel endet.

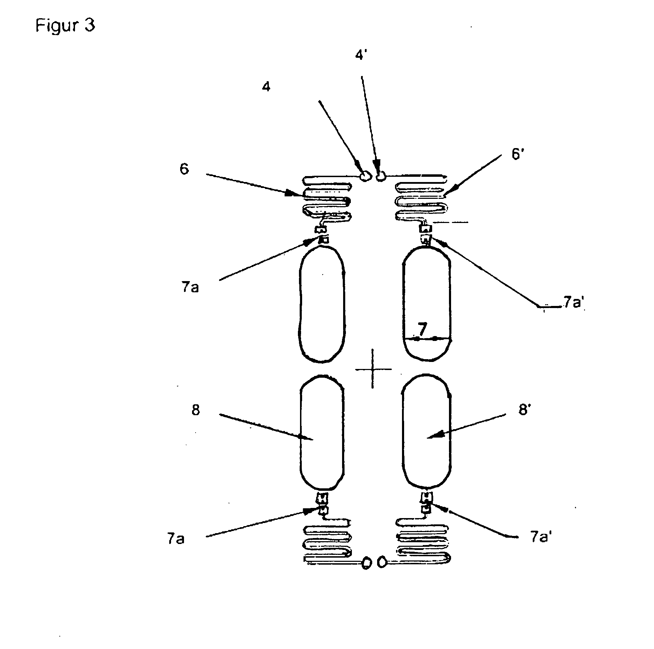

In Fig. 3 wird eine weitere beispielhafte Ausführungsform eines erfindungsgemäßen Trägerelements 1 gezeigt. Hier weist im Gegensatz zu der in Fig. 1 gezeigten Ausführungsform jede der Reaktionskammern 8, 8' einen separaten Zulauf 4, 4' auf. Diese separaten Zuläufe 4, 4' sind vorzugsweise in Fließrichtung nach dem Luer-Lok-Anschluss 11 angeordnet. Außerdem sind den Reaktionskammern 8, 8' im Anschluss an die Zuleitungskanäle 6, 6' anstelle von Einzelventilen die Doppelventile 7a, 7a' vorgeschaltet.

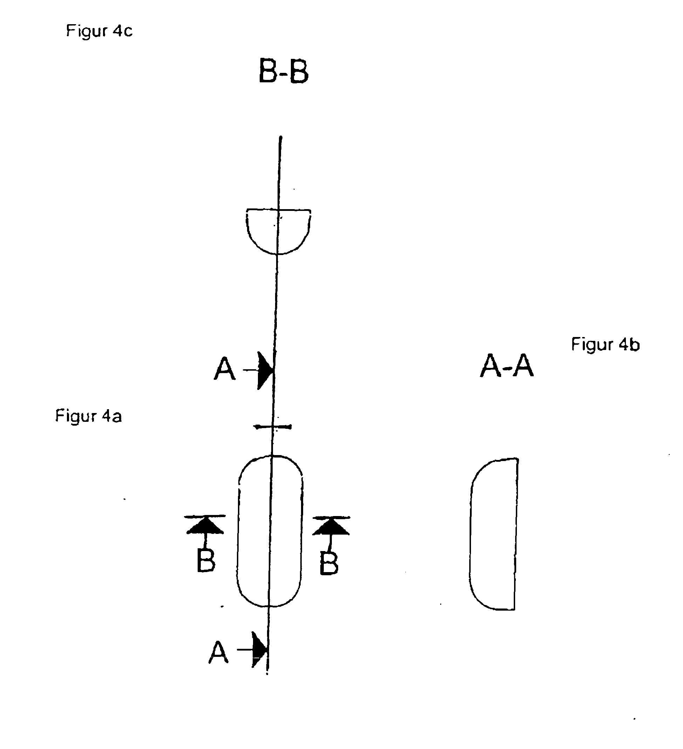

Die Figuren 4a - 4c zeigen eine bevorzugte Ausführungsform einer erfindungsgemäßen Reaktionskammer. Dabei zeigt Fig. 4a eine Draufsicht auf eine Reaktionskammer 8, 8'. In dieser Draufsicht erkennt man, dass die Übergange zwischen den Seitenwänden jeweils gekrümmt sind, d.h. keine rechten Winkel auftreten. Fig. 4b zeigt eine Querschnittsansicht entlang der Linie A - A in Fig. 4a und Fig. 4c eine Querschnittsansicht entlang der Linie B - B aus Fig. 4a. Hier kann man erkennen, dass auch die Übergänge zwischen den Seitenwänden und dem Boden gekrümmt sind und keine rechten Winkel aufweisen.

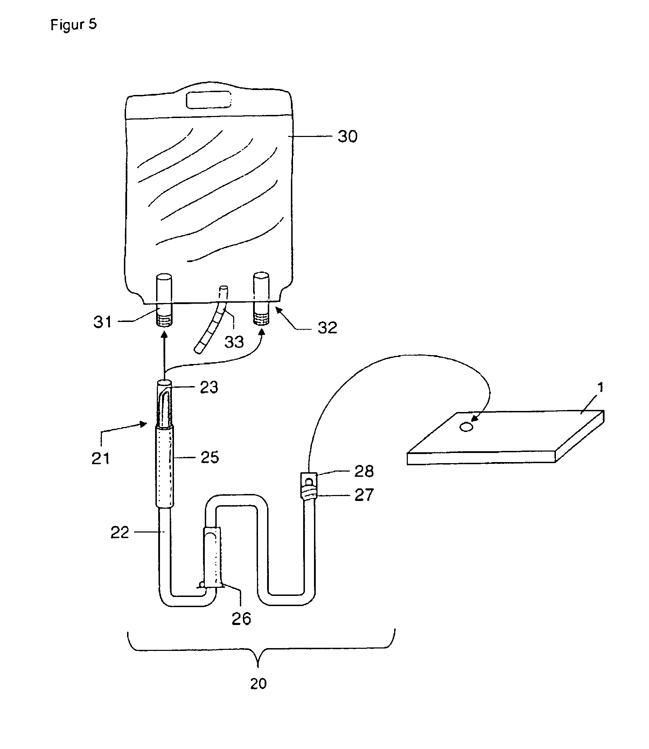

In Fig. 5 wird schließlich ein erfindungsgemäßes System mit Blutbeutel 30, Trägerelement 1 und Schlauchverbindung 20 gezeigt. An einem handelsüblichen Transfusionsbeutel 30 befinden sich standardmäßig zwei gleichartige Zugänge 31, 32, an denen das Transfusionsbesteck eingestochen werden kann. Es werden nie beide Zugänge 31, 32 für dieselbe Transfusion benötigt. Die Entscheidung, welcher der beiden Zugänge angestochen wird, liegt bei der Person, die das Transfusionsbesteck für die Transfusion anbringt. Die bevorzugte Ausführungsform des erfindungsgemäßen Systems zur Online-Kopplung des Trägerelements 1 mittels einer Schlauchverbindung 20 an den Transfusionsbeutel 30 bedient sich des Umstands, dass an einem Transfusionsbeutel 30 immer ein Zugang 31 oder 32 frei bleibt, der zum Anstechen mit einer sterilen Plastikkanüle geeignet ist. Die Schlauchverbindung 20 ist in dieser Variante ähnlich den handelsüblichen Transfusions- oder Infusionsbestecken, wie sie z. B. von den Firmen Terumo, Inframed, Hemomed und anderen erhältlich sind. Die Schlauchverbindung 20 besteht vorzugsweise aus folgenden Komponenten: Ein Schlauch 22, der an einem Ende eine Kunststoffkanüle 21 zum Einstechen in den Transfusionsbeutel 30 aufweist, die vor Benutzung mittels einer Schutzkappe 23 steril verschlossen ist. An die Kanüle 21 schließt sich eine Tropfkammer 25, die auch einen Blutfilter (nicht gezeigt) enthalten kann, an. Das Vorhandensein eines Blutfilters macht im Wesentlichen den Unterschied zwischen einem Transfusionsbesteck und einem Infusionsbesteck aus. Es handelt sich um Filter mit einer Porengröße von etwa 200 µm, die dazu dienen, möglicherweise im Blutbeutel 30 befindliche kleine Klumpen abzufiltern, bevor die Transfusion in die Venen des Patienten gelangt. Anschließend folgt ein Schlauch 22, der beispielsweise 10 bis 150 cm lang ist. Grundsätzlich sollte der Schlauch 22 so ausgestaltet sein, dass für den Test möglichst wenig Blut verbraucht wird. An dem Schlauch 22 ist eine Rollklemme 26 zur manuellen Dosierung des Blutflusses befestigt. Das zweite Schlauchende hat zum Abschluss einen Luer-, vorzugsweise einen Luer-Lok-Adaptor 27, der vor Gebrauch durch eine Schutzkappe 28 verschlossen ist. Der Luer-Lok-Adaptor 27 ist vorzugsweise als Male ausgebildet. Nach Entfernen der Schutzkappe 28 kann das Trägerelement 1 in einfacher Weise an den Luer-Adaptor der Schlauchverbindung 20 angesteckt bzw. an den Luer-Lok-Adaptor 27 der Schlauchverbindung 20 angeschraubt werden. Bei einem Male-Adaptor an der Schlauchverbindung 20 muss der korrespondierende Luer-Lok-Anschluss 11 auf dem Oberteil 3 des Trägerelements 1 als Female ausgebildet sein, und umgekehrt.

Wurde mit Hilfe der Schlauchverbindung 20 eine Verbindung zwischen Trägerelement 1 und Blutbeutel 30 hergestellt, wird durch Öffnen der Rollklemme 26 und den dadurch wirksamen hydrostatischen Druck das Blut bzw. Erythrozytenkonzentrat in die Kanäle des Trägerelements 1 eingebracht, bis in allen Reaktionskammern 8, 8' ein Bluttropfen erkennbar ist. Der Druck kann dabei manuell vom Bedienpersonal ausgeübt werden, es ist jedoch auch eine, innerhalb oder außerhalb der Schlauchverbindung angeordnete, zusätzliche Pumpvorrichtung denkbar. Die Schlauchverbindung 20 wird anschließend entfernt und das Trägerelement 1 mit der Hand in einer Weise bewegt, dass die Antikörper mit den korrespondierenden Antigenen auf den Erythrozyten reagieren können. Nach etwa 60 Sekunden wird in bekannter Weise auf Haemagglutinationsreaktion geprüft.

Die in Fig. 5 gezeigte Schlauchverbindung 20 ist eine vorgeschlagene Verbindung zusätzlich zum Transfusionsbesteck. Sie hat einen Stechmechanismus, wie er dem Fachmann blutbeutelseitig von den handelsüblichen Transfusionsbestecken her bekannt ist, um in den freien Zugang 31 oder 32 des Blutbeutels 30 eingestochen werden zu können. Das andere Ende ist ein Luer-Lok-Male-Adaptor 27, der mit dem Luer-Lok-Female-Anschluss 11 am Trägerelement 1 kompatibel ist. Auf diese Weise ist alles Material, das für einen Blutgruppentest notwendig ist, immer an Ort und Stelle. Die Vorteile dieser Variante liegen auf der Hand: Sie führt auf der Spenderseite zur vollständigen Vermeidung der Benutzung von Spritzenkanülen insbesondere im Rahmen einer Bestimmung der Blutgruppen A, B und O bzw. A, B, O und D am Patientenbett.

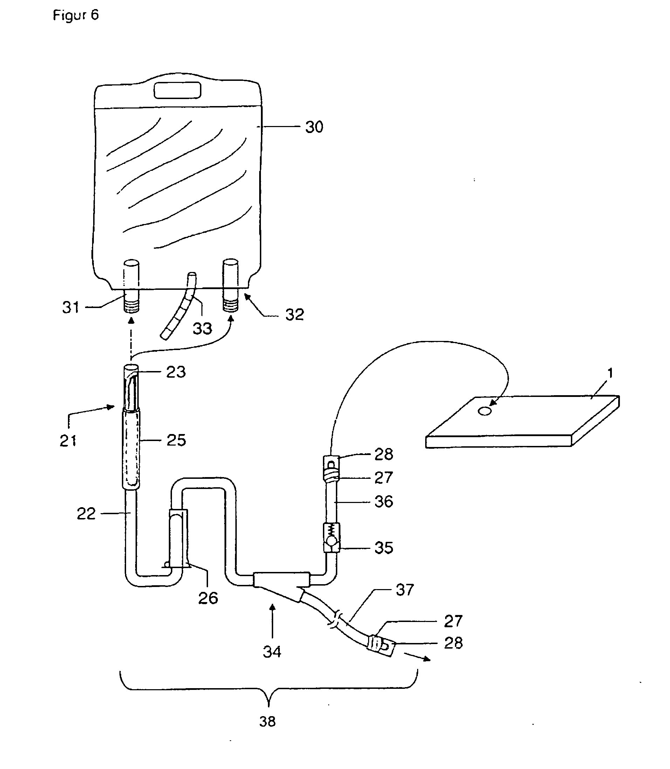

In Fig. 6 wird ein weiteres erfindungsgemäßes System mit Blutbeutel 30, Trägerelement 1 und Schlauchverbindung 38 gezeigt. Diese besonders bevorzugte Ausführungsform des erfindungsgemäßen Systems zur Online-Kopplung des Trägerelements 1 mittels einer Schlauchverbindung 38 an den Transfusionsbeutel 30 bedient sich eines Y-Verbindungsstückes 34, von dem ein Nebenschlauch 36 abzweigt, der zum Trägerelement 1 führt, und ein Hauptschlauch 37 abzweigt, der zum Patienten führt. Im Nebenschlauch 36 befindet sich eine Rückflusssperre 35, welche einen Rückfluss von Blut bzw. Erythrozytenkonzentrat in dem gegebenenfalls nicht-sterilen Nebenschlauch 36 in die sterilen Schlauchabschnitte 22, 37 verhindert. Die Schlauchverbindung 38 besteht ansonsten aus den in Figur 3 im Detail beschriebenen Komponenten.

Wenn mit Hilfe der Schlauchverbindung 38 eine Verbindung zwischen Trägerelement 1 und Blutbeutel 30 hergestellt wurde, kann wie bei der Schlauchverbindung 20 das Blut bzw. Erythrozytenkonzentrat in die Kanäle des Trägerelements 1 eingebracht werden. Hierbei fließt das Blut bzw. Erythrozytenkonzentrat durch den Schlauch 22 über die Verzweigung 34 und den Nebenschlauch 36 in das Trägerelement 1. Der Druck kann dabei manuell vom Bedienpersonal ausgeübt werden, es ist jedoch auch eine, innerhalb oder außerhalb der Schlauchverbindung angeordnete, zusätzliche Pumpvorrichtung denkbar. Die Schlauchverbindung 38 kann anschließend entfernt und das Trägerelement 1 mit der Hand in einer Weise bewegt werden, dass die Antikörper mit den korrespondierenden Antigenen auf den Erythrozyten reagieren können. Nach etwa 60 Sekunden wird in bekannter Weise auf Haemagglutinationsreaktion geprüft. Es ist außerdem möglich, das Trägerelement permanent am Blutbeutel hängen zu lassen, auch während und nach der Bestimmung des Patientenblutes. Eine solche Vorgehensweise kann insbesondere im Hinblick auf mögliche Verwechslungen von Vorteil sein.

Auch die in Fig. 6 gezeigte Schlauchverbindung 38 ist eine Variante eines handelsüblichen Transfusionsbestecks. Sie hat einen Stechmechanismus mit dem sie in den freien Zugang 31 bzw. 32 des Blutbeutels 30 eingestochen werden kann. Die beiden anderen Enden sind Luer-Lok-Male-Adaptoren 27, von denen einer mit dem Luer-Lok-Female-Anschluss 11 am Trägerelement 1 kompatibel ist. Der andere Luer-Lok-Male-Adaptor 27 wird verwendet, um das Blut bzw. Erythrozytenkonzentrat zu dem Patienten zu leiten. Auf diese Weise ist alles Material, das für einen Blutgruppentest notwendig ist, immer an Ort und Stelle. Hier bestehen auch die im Zusammenhang mit Fig. 5 aufgeführten Vorteile. Ein zusätzlicher Vorteil der in Figur 4 gezeigten besonders bevorzugten Variante besteht darin, dass der Blutbeutel selbst gemäss heutiger Praxis und Richtlinien nur einmal angestochen werden muss.

Im Stand der Technik werden Schlauchsegmente 33, die mit Blut bzw. Erythrozytenkonzentrat der zu transfundierenden Spende gefüllt sind, mittels einer Kanüle angestochen. Das so gewonnene Spenderblut bzw. Erythrozytenkonzentrat wird zur Blutgruppenbestimmung der Konserve (Spenderseite S des Trägerelements 1) verwendet. Es besteht hierbei ein erhebliches Kontaminations- und Infektionsrisiko für die Person, die das Segment 33 ansticht. Des Weiteren kommt es gerade in den Schlauchsegmenten 33 leichter als im Blutbeutel zu Verklumpungen von Zellen, was die Durchführung einer Blutgruppenbestimmung erschwert bzw. die Gefahr von Fehlbestimmungen erhöht. Bei beiden bevorzugten Varianten des erfindungsgemäßen Systems dagegen wird durch die Schlauchverbindung 20 ein komplett geschlossenes System zur Agglutinationsbestimmung auf der Spenderseite geschaffen. Somit wird hier das Kontaminations- und Infektionsrisiko für die Person, die den Test durchführt, praktisch ganz ausgeschlossen. Ein weiterer Vorteil der erfindungsgemäßen Schlauchverbindungen liegt in der Möglichkeit, dass das Krankenhauslabor bei der Vorbereitung der Bluttransfusion bereits im Labor sämtliche Komponenten bereitstellen kann, damit der Blutgruppentest bereits am Transfusionsbeutel hängt, wenn dieser ans Patientenbett kommt. Es wird dadurch die Möglichkeit ausgeschlossen, dass für den Blutgruppentest benötigte Komponenten am Ort der Transfusion nicht verfügbar sind.

Verwendet man statt der Schlauchverbindungen 20, 38 zur spenderseitigen Bestimmung eine auf eine handelsübliche Luer-Spritze aufgesetzte Kanüle, wird das Schlauchsegment 33 angestochen und das dort befindliche Blut oder Erythrozytenkonzentrat abgezogen. Darauf wird die Kanüle von der Spritze entfernt und die Spritze wird auf die Spritzenöffnung des Trägerelements 1 auf der Spenderseite S aufgesteckt (normale Luer-Spritzen) bzw. durch Drehung im Uhrzeigersinn aufgeschraubt (Luer-Lok-Spritzen). Dann wird durch leichten Druck das Blut oder Erythrozytenkonzentrat in die Kanäle des Trägerelements 1 eingespritzt bis in allen Reaktionskammern 8, 8' ein Bluttropfen erkennbar ist. Die Spritze wird entfernt und das Trägerelement mit der Hand in der Weise bewegt, dass die Antikörper mit den korrespondierenden Antigenen auf den Erythrozyten reagieren können. Nach etwa 60 Sekunden wird in der dem Fachmann bekannten Weise auf Haemagglutinationsreaktion geprüft.

Zur empfängerseitigen Bestimmung wird mit einer handelsüblichen Luer-Spritze oder Luer-Lok-Spritze am Patientenzugang Venenblut oder Kapillarblut abgezogen. Darauf wird die Spritze auf die Spritzenöffnung des Trägerelements 1 auf der Empfängerseite E aufgesteckt (normale Luer-Spritzen) bzw. durch Drehung im Uhrzeigersinn aufgeschraubt (Luer-Lok-Spritzen). Dann wird durch leichten Druck das Empfängerblut in die Kanäle des Trägerelements eingespritzt, bis in allen Reaktionskammern ein Bluttropfen erkennbar ist. Die Spritze wird entfernt und das Trägerelement mit der Hand in der Weise bewegt, dass die Antikörper mit den korrespondierenden Antigenen auf den Erythrozyten reagieren können. Nach etwa 60 Sekunden wird wiederum in der dem Fachmann bekannten Weise auf Haemagglutinationsreaktion geprüft.

Selbstverständlich können mit Hilfe der Erfindung auch andere Tests, die auf ähnlichen Prinzipien wie bei der Blutgruppenbestimmung beruhen, ausgeführt werden, wie zum Beispiel Partikel-Agglutinationstests.

## Patentansprüche

1. Trägerelement (1) für diagnostische Tests, insbesondere zur Agglutinations-bestimmung, mit mindestens einem Unterteil (2), mindestens einem Oberteil (3) und mindestens einem zentralen Zulauf (4), wobei von dem mindestens einen zentralen Zulauf (4) mindestens zwei Zuleitungskanäle (6, 6') zu jeweils einer Reaktionskammer (8, 8') führen,
**dadurch gekennzeichnet, dass**
die mindestens zwei Zuleitungskanäle (6, 6') jeweils gleiche Volumina aufweisen und dass jeweils passive Kapillarventile den Reaktionskammern (8, 8') direkt vorgeschaltet sind und wobei die Zuleitungskanäle (6, 6') von der gleichen Stelle (5) des zentralen Zulaufs (4) abzweigen.

2. Trägerelement (1) nach dem vorhergehenden Anspruch, **dadurch gekennzeichnet, dass** die mindestens zwei Zuleitungskanäle (6, 6') sowohl die gleiche Länge als auch den gleichen Durchmesser aufweisen.

3. Trägerelement (1) nach einem der vorhergehenden Ansprüche, **dadurch gekennzeichnet, dass** mindestens einer der Zuleitungskanäle (6, 6') mäanderförmig ausgebildet ist.

4. Trägerelement (1) nach einem der vorhergehenden Ansprüche, **dadurch gekennzeichnet, dass** die Reaktionskammern (8, 8') jeweils einen Lüftungskanal (10, 10') aufweisen.

5. Trägerelement (1) nach dem vorhergehenden Anspruch, **dadurch gekennzeichnet, dass** jeweils zwischen Reaktionskammer (8, 8') und Lüftungskanal (10, 10') eine Ventilfunktion (9, 9') angeordnet ist.

6. Trägerelement (1) nach einem der vorhergehenden Ansprüche, **dadurch gekennzeichnet, dass** das Oberteil (3) über den Reaktionskammern (8, 8') jeweils mindestens eine Öffnung aufweist.

7. Trägerelement (1) nach dem vorhergehenden Anspruch, **dadurch gekennzeichnet, dass** die mindestens eine Öffnung einen Durchmesser von 1,5 mm oder weniger aufweist.

8. Trägerelement (1) nach einem der vorhergehenden Ansprüche, **dadurch gekennzeichnet, dass** das Unterteil (2) und das Oberteil (3) des Trägerelements (1) dicht verbunden sind.

9. Trägerelement (1) nach einem der vorhergehenden Ansprüche, **dadurch gekennzeichnet, dass** die Reaktionskammern (8, 8') mit Antikörpern oder Antigenen beschickt sind.

10. Trägerelement (1) nach einem der vorhergehenden Ansprüche, **dadurch gekennzeichnet, dass** die Reaktionskammern (8, 8') mit Antikörpern oder Antigenen zur Agglutinationsbestimmung beschickt sind.

11. Trägerelement (1) nach dem vorhergehenden Anspruch, **dadurch gekennzeichnet, dass** die Antikörper oder Antigene in den Reaktionskammern (8, 8') in gelöster, lyophilisierter oder an eine Festphase gebundener Form vorliegen.

12. Trägerelement (1) nach einem der vorhergehenden Ansprüche, **dadurch gekennzeichnet, dass** in der Reaktionskammer eine Lösung vorliegt, die aus einer Lösung der Antikörper oder Antigene und einer Lösung mit höherer Dichte gebildet wird.

13. Trägerelement (1) nach dem vorhergehenden Anspruch, **dadurch gekennzeichnet, dass** als Lösung mit höherer Dichte Glycerin verwendet wird.

14. Trägerelement (1) nach dem vorhergehenden Anspruch, **dadurch gekennzeichnet, dass** das Glycerin in einer Konzentration zwischen 5 und 30 % (w/v) vorliegt.

15. Trägerelement (1) nach einem der vorhergehenden Ansprüche, **dadurch gekennzeichnet, dass** die Reaktionskammern (8, 8') Polymerpartikel enthalten, die mit Liganden beschichtet sind.

16. Trägerelement (1) nach einem der vorhergehenden Ansprüche, **dadurch gekennzeichnet, dass** die Reaktionskammern (8, 8') des Trägerelements (1) räumlich derart angeordnet sind, dass sie mit handelsüblichen automatischen Pipettierstationen beschickt werden können.

17. Trägerelement (1) nach einem der vorhergehenden Ansprüche, **dadurch gekennzeichnet, dass** an dem mindestens einen zentralen Zulauf (4) ein Luer- oder Luer-Lok-Anschluss (11) anordenbar sind.

18. Trägerelement (1) nach einem der vorhergehenden Ansprüche, **dadurch gekennzeichnet, dass** die Reaktionskammern (8, 8') eine Badewannenform aufweisen.

19. Trägerelement (1) nach einem der vorhergehenden Ansprüche, **dadurch gekennzeichnet, dass** eine Testflüssigkeit mit Hilfe von Druckbeaufschlagung von dem Zulauf (4) zu den Reaktionskammern (8, 8') befördert wird.

20. System mit einem Trägerelement (1) nach einem der Ansprüche 1 bis 19, einem Blutbeutel (30) und einer Schlauchverbindung (20), bei dem mit der Schlauchverbindung (20) eine Verbindung zwischen dem Trägerelement (1) und dem Blutbeutel (30) hergestellt wird, wobei die Schlauchverbindung (20) eine Kunststoffkanüle (21) an ihrem blutbeutelseitigen Ende, einen Luer- oder Luer-Lok-Adaptor (27) an ihrem trägerelementseitigen Ende, und einen Schlauch (22) zwischen den beiden Enden, aufweist

21. System mit einem Trägerelement (1) nach einem der Ansprüche 1 bis 19, einem Blutbeutel (30) und einer Schlauchverbindung (38), wobei die Schlauchverbindung (38) einen Schlauch (22) mit einer Verzweigung (34) aufweist, an die sich ein Nebenschlauch (36) und ein Hauptschlauch (37) anschließt und wobei der Nebenschlauch (36) einen Anschluss für ein Trägerelement (1) aufweist.

## Claims

1. Supporting element (1) for diagnostic tests, in particular for determination of agglutination, with at least one lower part (2), at least one upper part (3) and at least one central intake (4), whereby from the at least one central intake (4) at least two supply channels (6, 6') respectively lead to a reaction chamber (8, 8'),
**characterized in that**
the at least two supply channels (6, 6') respectively comprise the same volumes, and **in that** respectively passive capillary valves are connected directly upstream to the reaction chambers (8, 8'), and whereby the supply channels (6, 6') branch off from the same position (5) of the central intake (4).

2. Supporting element (1) according to the preceding claim, **characterized in that** the at least two supply channels (6, 6') comprise both the same length as well as also the same diameter.

3. Supporting element (1) according to one of the preceding claims, **characterized in that** at least one of the supply channels (6, 6') is formed in a meandering pattern.

4. Supporting element (1) according to one of the preceding claims, **characterized in that** the reaction chambers (8, 8') respectively comprise a venting channel (10, 10').

5. Supporting element (1) according to the preceding claim, **characterized in that** a valve function (9, 9') is arranged respectively between the reaction chamber (8, 8') and the venting channel (10, 10').

6. Supporting element (1) according to one of the preceding claims, **characterized in that** the upper part (3) above the reaction chambers (8, 8') respectively comprises at least one opening.

7. Supporting element (1) according to the preceding claim, **characterized in that** the at least one opening comprises a diameter of 1,5 mm or less.

8. Supporting element (1) according to one of the preceding claims, **characterized in that** the lower part (2) and the upper part (3) of the supporting element (1) are tightly connected.

9. Supporting element (1) according to one of the preceding claims, **characterized in that** the reaction chambers (8, 8') are loaded with antibodies or antigenes.

10. Supporting element (1) according to one of the preceding claims, **characterized in that** the reaction chambers (8, 8') are loaded with antibodies or antigenes for determination of agglutination.

11. Supporting element (1) according to the preceding claim, **characterized in that** the antibodies or antigenes are provided in the reaction chambers (8, 8') in dissolved form, lyophilized form or in a form bound to a solid phase.

12. Supporting element (1) according to one of the preceding claims, **characterized in that** a solution is provided in the reaction chamber, which is formed by a solution of the antibodies or antigenes and a solution with higher density.

13. Supporting element (1) according to the preceding claim, **characterized in that** glycerin is used as solution with higher density.

14. Supporting element (1) according to the preceding claim, **characterized in that** the glycerin is provided at a concentration in between 5 and 30% (w/v).

15. Supporting element (1) according to one of the preceding claims, **characterized in that** the reaction chambers (8, 8') contain polymer particles, which are coated with ligands.

16. Supporting element (1) according to one of the preceding claims, **characterized in that** the reaction chambers (8, 8') of the supporting element (1) are spatially arranged such that they can be loaded with commercially available automatic pipetting stations.

17. Supporting element (1) according to one of the preceding claims **characterized in that** a luer or a luer-lok-connector (11) is arrangeable at the at least one central intake (4).

18. Supporting element (1) according to one of the preceding claims, **characterized in that** the reaction chambers (8, 8') comprise a bath tub shape.

19. Supporting element (1) according to one of the preceding claims, **characterized in that** a test liquid is conveyed from the intake (4) to the reaction chambers (8, 8') making use of application of pressure.

20. System with a supporting element (1) according to one of the claims 1 to 19, with a blood bag (30) and a tube connection (20), whereby, by means of the tube connection (20) a connection between the supporting element (1) and the blood bag (30) is established, whereby the tube connection (20) comprises a plastic cannula (21) at its blood bag sided end, a luer- or luer-lok adapter (27) at its support element sided end and a tube (22) in between the two ends.

21. System with a supporting element (1) according to one of the claims 1 to 19, a blood bag (30) and a tube connection (38), whereby the tube connection (38) comprises a tube (22) with a branch (34) at which a side tube (36) and a main tube (37) is connected and whereby the side tube (36) comprises a connection for a supporting element (1).

## Revendications

1. Elément support (1) pour tests diagnostiques, en particulier pour détermination de l'agglutination, comportant au moins une partie inférieure (2), au moins une partie inférieure (3) et au moins une prise centrale (4), au moins deux canaux d'alimentation (6, 6') conduisant, à partir de la ou des prises centrales (4), chacun à au moins une chambre de réaction (8, 8'), **caractérisé en ce que** les au moins deux canaux d'alimentation (6, 6') ont chacun le même volume, et que des vannes capillaires passives sont montées directement en amont de chaque chambre de réaction (8, 8'), les canaux d'alimentation (6, 6') partant du même point (5) de la prise centrale (4).

2. Elément support (1) selon la revendication précédente, **caractérisé en ce que** les au moins deux canaux d'alimentation (6, 6') ont la même longueur, ainsi que le même diamètre.

3. Elément support (1) selon l'une des revendications précédentes, **caractérisé en ce qu'**au moins l'un des canaux d'alimentation (6, 6') est configuré en forme de méandre.

4. Elément support (1) selon l'une des revendications précédentes, **caractérisé en ce que** chaque chambre de réaction (8, 8') comprend un canal d'aération (10, 10').

5. Elément support (1) selon la revendication précédente, **caractérisé en ce qu'**un organe à fonction de vanne (9, 9') est disposé entre chaque chambre de réaction (8, 8') et chaque canal d'aération (10, 10').

6. Elément support (1) selon l'une des revendications précédentes, **caractérisé en ce que** la partie supérieure (3) située au-dessus de chacune des chambres de réaction (8, 8') comprend au moins une ouverture.

7. Elément support (1) selon la revendication précédente, **caractérisé en ce que** la ou les ouvertures ont un diamètre de 1,5 mm ou moins.

8. Elément support (1) selon l'une des revendications précédentes, **caractérisé en ce que** la partie inférieure (2) et la partie supérieure (3) de l'élément support (1) sont assemblées l'une à l'autre d'une manière étanche.

9. Elément support (1) selon l'une des revendications précédentes, **caractérisé en ce que** les chambres de réaction (8, 8') sont alimentées en anticorps ou en antigènes.

10. Elément support (1) selon l'une des revendications précédentes, **caractérisé en ce que** les chambres de réaction (8, 8') sont alimentées en anticorps ou antigènes pour la détermination de l'agglutination.

11. Elément support (1) selon la revendication précédente, **caractérisé en ce que** les anticorps ou les antigènes sont présents dans les chambres de réaction (8, 8') sous forme dissoute, sous forme lyophilisée ou sous une forme fixée à une phase solide.

12. Elément support (1) selon l'une des revendications précédentes, **caractérisé en ce que** la chambre de réaction contient une solution qui est formée à partir d'une solution des anticorps ou des antigènes et d'une solution ayant une masse volumique plus élevée.

13. Elément support (1) selon la revendication précédente, **caractérisé en ce que**, en tant que solution ayant une masse volumique plus élevée, on utilise du glycérol.

14. Elément support (1) selon la revendication précédente, **caractérisé en ce que** le glycérol est présent à une concentration comprise entre 5 et 30 % (p/v).

15. Elément support (1) selon l'une des revendications précédentes, **caractérisé en ce que** les chambres de réaction (8, 8') contiennent des particules polymères revêtues de ligands.

16. Elément support (1) selon l'une des revendications précédentes, **caractérisé en ce que** les chambres de réaction (8, 8') de l'élément support (1) sont spatialement disposées de façon à pouvoir recevoir des stations automatiques de pipetage du commerce.

17. Elément support (1) selon l'une des revendications précédentes, **caractérisé en ce qu'**un embout Luer ou Luer-Lock (11) peut être disposé contre la ou les prises centrales (4).

18. Elément support (1) selon l'une des revendications précédentes, **caractérisé en ce que** les chambres de réaction (8, 8') ont la forme d'une baignoire.

19. Elément support (1) selon l'une des revendications précédentes, **caractérisé en ce qu'**un liquide d'essai est refoulé de la prise (4) vers les chambres de réaction (8, 8'), par application d'une pression.

20. Système comportant un élément support (1) selon l'une des revendications 1 à 19, une poche à sang (30) et un raccord (20) pour flexibles, dans lequel un raccordement entre l'élément support (1) et la poche à sang (30) est réalisé par le raccord (20) pour flexibles, le raccord (20) pour flexibles comportant une canule plastique (21) en son extrémité côté poche à sang, un adaptateur Luer ou Luer-Lok (27) en son extrémité côté élément support, et un tuyau flexible (22) entre les deux extrémités.

21. Système comportant un élément support (1) selon l'une des revendications 1 à 19, une poche à sang (30) et un raccord (38) pour flexibles, le raccord (38) pour flexibles comportant un flexible (22) présentant une bifurcation (34), à laquelle sont raccordés un flexible auxiliaire (36) et un flexible principal (37), le flexible auxiliaire (36) comportant une prise de raccordement pour un élément support (1).
